(19) Europäisches Patentamt
European Patent Office
Office européen des brevets

(11) EP 0 995 426 A2

(12) **EUROPÄISCHE PATENTANMELDUNG**

(43) Veröffentlichungstag:
**26.04.2000 Patentblatt 2000/17**

(51) Int. Cl.[7]: **A61K 7/42**

(21) Anmeldenummer: **99109658.7**

(22) Anmeldetag: **15.05.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **30.05.1998 DE 19824418**

(71) Anmelder: **Goldschmidt AG**
**45127 Essen (DE)**

(72) Erfinder:
- **Bungard, Andrea, Dr.**
  **45136 Essen (DE)**
- **Jenni, Klaus, Dr.**
  **58454 Witten (DE)**
- **Leidreiter, Holger, Dr.**
  **45529 Hattingen (DE)**
- **Walter, Alfred**
  **45239 Essen (DE)**

(54) **Transparente Sonnenschutzgele**

(57) Gegenstand der Erfindung sind transparente Sonnenschutzgele enthaltend Silikonöl, Polyoxyalkylen-Organopolysiloxane, Wasser, eine Komponente ausgewählt aus Alkoholen und/oder Polyolen und wenigstens einen in der wäßrigen oder in der öligen Phase löslichen UV-Absorber.

Die transparenten Sonnenschutzgele enthalten

(a) Silikonöl
(b) Polyoxyalkylen-Organopolysiloxane der allgemeinen Formel (I)

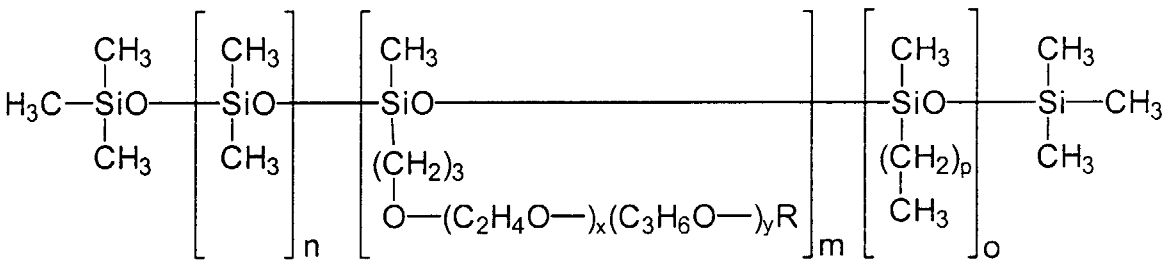

(I)

wobei

R ein Alkylrest mit 1 bis 4 Kohlenstoffatomen oder ein Wasserstoffrest ist,
n = 10 bis 200,
m = 1 bis 25 und
o = 1 bis 100 mit der Maßgabe ist, daß im durchschnittlichen Molekül o ≥ m und 3 o < n ist,
p = 7 bis 17 ist, und

die Molmasse des Restes $(C_2H_4O\text{-})_x\text{-}(C_3H_6O\text{-})_yR$ 250 bis 2000 beträgt, wobei x und y so gewählt sind, daß das Gewichtsverhältnis von Oxyethylen- zu Oxypropylengruppen 100 : 0 = bis 20 : 80 beträgt,
(c) Wasser,
(d) eine Komponente ausgewählt aus Alkoholen und/oder Polyolen und
(e) wenigstens einen in der wäßrigen oder in der öligen Phase löslichen UV-Absorber.

EP 0 995 426 A2

**Beschreibung**

Gegenstand der Erfindung sind transparente Sonnenschutzgele enthaltend Silikonöl, Polyoxyalkylen-Organopolysiloxane, Wasser, eine Komponente ausgewählt aus Alkoholen und/oder Polyolen und wenigstens einen in der wäßrigen oder in der öligen Phase löslichen UV-Absorber.

Transparente Polyol und Wasser/Silikon enthaltende Emulsionen sind bekannt und werden beispielsweise als Antitranspirantien oder Haargele verwendet. Durch Anpassung des Brechungsindexes der Wasserphase an den der Ölphase mit Hilfe von Polyolen wie Glycerin, Polyglycerin, Propylenglykol, Hexylenglykol und anderen Polyolen werden transparente Emulsionen, sogenannte Gele erhalten. Diese Produkte werden üblicherweise mit Silikon-Emulgatoren hergestellt, wobei die Ölphase hauptsächlich Cyclomethicone (cyclische Polydimethylsiloxane) oder niedermolekulare Silikonöle (lineare Polydimethylsiloxane) enthält.

EP 0 175 884 B1 beschreibt die Verwendung von Polyoxyalkylen-Siloxan-Copolymerisaten mit an Siliciumatomen gebundenen langkettigen Alkylresten als Emulgatoren zur Herstellung von W/O-Emulsionen. Diese Patentschrift betrifft insbesondere die Verwendung von Copolymerisaten der durchschnittlichen Formel (I)

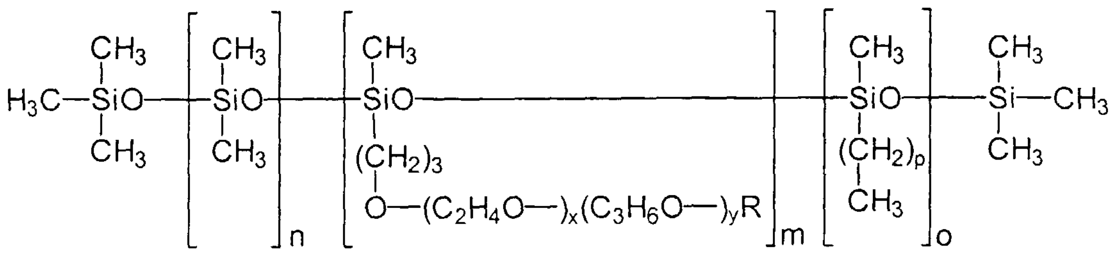

(I)

wobei

R ein Alkylrest mit 1 bis 4 Kohlenstoffatomen oder ein Wasserstoffrest ist,
n = 10 bis 200,
m = 1 bis 25 und
o = 1 bis 100 mit der Maßgabe ist, daß im durchschnittlichen Molekül o ≥ m und 3 o < n ist,
p = 7 bis 17 ist, und

die Molmasse des Restes $(C_2H_4O-)_x-(C_3H_6O-)_yR$ 250 bis 2000 beträgt, wobei x und y so gewählt sind, daß das Gewichtsverhältnis von Oxyethylen- zu Oxypropylengruppen 100 : 0 bis 20 : 80 beträgt,
als Emulgatoren zur Herstellung von W/O-Emulsionen, deren ölige Phase aus Silikonöl besteht oder dieses enthält, in Mengen von 0,3 bis 5 Gew.-%, bezogen auf das Gesamtgewicht der Emulsion.

Ähnliche $\alpha,\overline{\omega}$-Polyetherpolysiloxane sind als W/O-Emulgatoren aus der EP 0 819 426 A2 bekannt. Diese Patentanmeldung betrifft die Verwendung von Copolymerisaten der allgemeinen Formel (II)

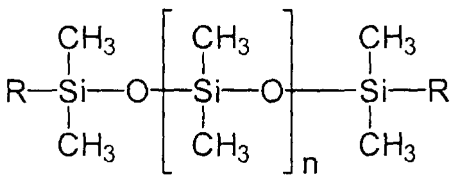

(II)

wobei

$R = -(CH_2-)_mO-(C_2H_4O-)_x(C_3H_6O-)_yR^1$
m = 2 bis 4,
x = 3 bis 100,
y = 0 bis 50

$R^1$ = H, $CH_3$,$CH_2CH_3$,
n = 50 bis 200,

als Emulgator in W/O Emulsionen in Mengen von 0,1 bis 20 Gew.-%, bezogen auf das Gesamtgewicht der Emulsion.

Weiterhin sind aus der EP 0 558 102 A1 Silikongelzusammensetzungen bekannt, die

(a) ein Silikonöl,
(b) ein Polyoxyalkylen-Organopolysiloxan der allgemeinen Formel (III)

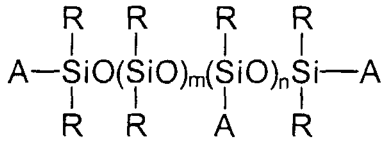

(III)

wobei

R für eine Methyl- oder Phenylgruppe steht,
A ausgewählt ist aus der Gruppe bestehend aus Methyl, Phenyl oder Poloxyalkylen-Gruppen der Formel

$$-C_3H_6O(C_2H_4O)_a(C_3H_6O)_bR',$$

wobei R' ausgewählt ist aus der Gruppe bestehend aus Wasserstoff, einer Acylgruppe oder einer Alkylgruppe mit 1 bis 4 Kohlenstoffatomen,
a eine ganze Zahl im Bereich von 5 bis 50
b eine ganze Zahl im Bereich von 5 bis 50,
m eine ganze Zahl im Bereich von 50 bis 1000 und
n eine ganze Zahl im Bereich von 1 bis 40 ist und

(c) Wasser enthalten.

Konventionelle Sonnenschutzgele, sogenannte Balme sind trübe, emulsionsartig aussehende Hydrogele auf Basis von Polyacrylaten oder von Hydrocolloiden, wie Cellulosederivaten oder Xanthan Gum, meist in Kombination mit Polyacrylaten, deren alkylmodifizierte Derivate eine gewisse Emulgierwirkung aufweisen.

Im Beispiel 1 der EP 0 568 102 A1 wird ein Sonnenschutzgel beschrieben, das ein Silikonöl, ein Organopolysiloxan, Wasser sowie ein Gewichtsteil eines UV-Absorbers Escalol®507 enthält. Hierbei handelt es sich um einen nichtwasserlöslichen, öllöslichen Lichtschutzfilter, nämlich um Octyldimethyl PABA. Die äußerst geringe Menge des Lichtschutzfilters bewirkt eine nur geringe Sonnenschutzwirkung. Im Handel erhältlich von Johnson & Johnson ist das Produkt Piz Buin Classic Brown Hydro Gel mit folgender Zusammensetzung: Wasser, Cyclomethicon (und) Dimethiconcopolyol, Glycerin, Alkohol, Propylenglycol, Phenylbenzimidazolsulfonsäure (wasserlöslicher Filter) , Tetrahydroxypropylethylendiamin, Benzophenon-4 (wasserlöslicher UV-Filter), Duft- und Aromastoffe, Hyaluronsäure, Natiumhydroxid, C.I. 15510, C.I. 47005 (gelbbraune Farbe).

Dieses Produkt entspricht einer transparenten W/Si-Emulsion. Die Konsistenz ist dickflüssig; die Emulsion(als Hydrogel bezeichnet) ist in braunen Quetschtuben erhältlich. Es werden keine kosmetischen Öle (Esteröle, Triglyceride) und ausschließlich wasserlösliche Filter verwendet. Der Emulgator „Cyclomethicon (und) Dimethiconcopolyol" entspricht dem Wettbewerbsprodukt DC 3225 C von Dow Corning und ist nicht in der lage, die oben erwähnten Öle oder öllöslichen Filter zu stabilisieren. Dieses Beispiel hat einen Lichtschutzfaktor 8 nach DIN.

Wird jedoch die Menge des Lichtschutzfilters erhöht, so tritt eine gravierende Änderung des Brechungsindexes auf, so daß das Gel die gewünschte Transparenz verliert.

Dementsprechend besteht die Aufgabe der vorliegenden Erfindung darin, transparente Sonnenschutzgele zur Verfügung zu stellen, die eine hohe Lichtschutzwirkung entfalten. Die erfindungsgemäßen Sonnenschutzgele sollten darüber hinaus frei von Polyacrylaten sein.

Erfindungsgemäß wurde gefunden, daß die vorgenannte Aufgabe gelöst werden kann, durch transparente Sonnenschutzgele, enthaltend Silikonöl, Polyoxyalkylen-Organopolysiloxane, Wasser, eine Komponente ausgewählt aus Alkoholen und/oder Polyolen und wenigstens einen in der wäßrigen oder in der öligen Phase löslichen UV-Absorber.

Dementsprechend betrifft die vorliegende Erfindung in einer ersten Ausführungsform transparente Sonnenschutzgele, enthaltend

(a) Silikonöl
(b) Polyoxyalkylen-Organopolysiloxane der allgemeinen Formel (I)

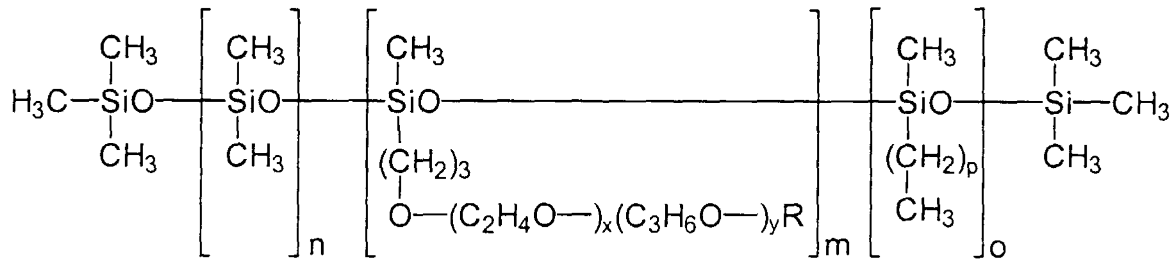

(I)

wobei

R ein Alkylrest mit 1 bis 4 Kohlenstoffatomen oder ein Wasserstoffrest ist,
n = 10 bis 200,
m = 1 bis 25 und
o = 1 bis 100 mit der Maßgabe ist, daß im durchschnittlichen Molekül o ≥ m und 3 o < n ist,
p = 7 bis 17 ist und

die Molmasse des Restes $(C_2H_4O-)_x-(C_3H_6O-)_yR$ 250 bis 2000 beträgt, wobei x und y so gewählt sind, daß das Gewichtsverhältnis von Oxyethylen- zu Oxypropylengruppen 100 : 0 bis 20 : 80 beträgt,
(c) Wasser
(d) eine Komponente ausgewählt aus Alkoholen und/oder Polyolen und
(e) wenigstens einen in der wäßrigen oder in der öligen Phase löslichen UV-Absorber.

Überraschenderweise zeigen die so erhaltenen Sonnenschutzemulsionen, die aufgrund ihres transparenten Aussehens und der bei Raumtemperatur festen Konsistenz als Gele bezeichnet werden, eine Reihe erstaunlicher Eigenschaften:

Die erfindungsgemäßen Gele stellen eine neue Produktform von Sonnenschutz dar. Es stehen erstmals völlig transparente Sonnenschutzformulierungen mit Lichtschutzfaktoren von 9 bis 20, bestimmt nach Colipa („Die Methode zur Bestimmung des Lichtschutzfaktors", IKW Broschüre, 1. Auflage, Juli 1995) mit pastöser/gelartiger Konsistenz zur Verfügung. Die erfindungsgemäßen Gele lassen sich in beliebige Behälter verpacken, wobei besonders geeignete Verpackungsformen, Tuben oder Roll-on-Spender sind. Die Einstellung des Brechungsindexes geschieht hierbei in an sich bekannter Weise. Bedingt durch hohe Konzentration an Lichtschutzfilter in der öligen Phase, zeigen die erfindungsgemäßen Gele durch Wechselwirkung mit dem Licht interessante Farberscheinungen, die je nach Rezepturzusammensetzung und Blickwinkel variieren. Überraschenderweise wurde gefunden, daß die Emulsionen, deren Ölphase, vorwiegend aus Silikonölen und öllöslichen UV-Filter besteht, Langzeit- und Temperatur-stabil sind, da üblicherweise in Sonnenschutzemulsionen kosmetische Öle zur Solubilisierung der Filter verwendet werden. Bei in-vivo- und in-vitro-Lichtschutzfaktorbestimmungen von erfindungsgemäßen Gelen, die wasserlösliche UV-Absorber enthalten, wurden erstaunlich hohe Werte gemessen, die über denen konventioneller W/O- und O/W-Emulsionen liegen.

Im Gegensatz zu konventionellen Sonnenschutzgelen, die trübe Hydrogele bzw. emulgatorfreie Balmformulierungen sind, handelt es sich bei den erfindungsgemäßen Gelen um echte Emulsionen mit disperser Polyol/Wasserphase. Der Vorteil der Alkohol/Polyol+W/Si+Öl-Emulsionsstruktur ist, daß der synergistische Effekt von wasserlöslichen und öllöslichen Filtern genutzt werden kann. In die erfindungsgemäßen transparenten Gele können weiterhin auch kosmetische Öle eingearbeitet werden, sofern der Brechungsindex der Formulierung durch Verwendung an Alkoholen/Polyolen einstellbar ist. Hierzu sollte der Brechungsindex der eingesetzten Öle möglichst niedrig liegen. Die Menge der Öle beträgt vorzugsweise 0,5 bis 5 Gew.-%, bezogen auf die Gesamtzusammensetzung.

Die erfindungsgemäßen transparenten Emulsionen sind, bedingt durch die Emulsionsform mit kohärenter Ölphase und Verwendung von Silikon-Emulgatoren, im Gegensatz zu den konventionellen Hydrogelen wasserfest. Der Zusatz von flüssigen Organopolysiloxanen zu externen Phasen erlaubt eine weitere Verbesserung der Haftung der Filtersubstanzen auf der Haut und somit eine Verbesserung gegenüber bestehenden Balmen oder Hydrogelen. Somit

werden auch nichtklebrige Gele erhältlich.

Besonders bevorzugt im Sinne der vorliegenden Erfindung sind die Silikonöle ausgewählt aus niedrig- bis hochviskosen Diorgano-Polysiloxanen wie Dimethyl -Polysiloxanen, Methylphenyl-Polysiloxanen und Dimethylsiloxan-Methylphenylsiloxancopolymeren, cyclischen Siloxanen wie Octamethyl-Cyclotetracyclosiloxan, Decamethyl -Cyclopentasiloxan und Tetramethyl-Tetraphenyl-Tetracyclosiloxan; Lösungen von cyclischen Siloxanen mit hoher Molmasse, von Dimethylpolysiloxan-Gumen, Dimethylsiloxan-Methylphenylsiloxan-Copolymer-Gumen und Dimethylpolysiloxan-Gumen; TrimethylsiloxyKieselsäuren und cyclischen Siloxanlösungen von Trimethylsiloxy-Kieselsäuren, Diorgano-Polysiloxanen mit $C_6$ bis $C_{50}$ Alkylgruppen; und Aminogruppen enthaltenden Diorgano-Polysiloxanen. Die Silikonöle können in den erfindungsgemäßen Gelen allein oder in Kombination mehrerer Komponenten enthalten sein. Insbesondere bevorzugt sind Polysiloxan-Polyalkylen-Copolymere und Dialkoxydimethylpolysiloxan.

Der Gehalt an Silikonöl ist in den erfindungemäßen Gelen nicht besonders eingeschränkt, jedoch wird Silikonöl vorzugsweise in einer Menge 10 bis 80 Gew.-%, bezogen auf die Gesamtzusammensetzung eingesetzt.

Die Polyoxyalkylen-Organopolysiloxane der allgemeinen Formel (I) dienen als Emulgator. Die Menge der Polyoxyalkylengruppen in der Komponente (b) ist nicht besonders eingeschränkt, wobei jedoch ein bevorzugter Gehalt der Polyoxyalkylengruppen in den Bereich von 20 bis 70 Gew.-% fällt. Im übrigen wird zur Beschreibung der Komponente (b) voll inhaltlich auf die eingangs erwähnte EP 0 818 426 A2 Bezug genommen. Selbstverständlich können auch Gemische von Polyoxyalkylen-Organosiloxanen eingesetzt werden.

Die Komponente (b) ist weder hinsichtlich des Molekulargewichts noch der Viskosität bei 25 °C besonders eingeschränkt. Insbesondere dann nicht, wenn diese zu stabilen Gelen führen, die ein trockenes Hautgefühl vermitteln.

Der Gehalt der Komponente (b) in den erfindungsgemäßen Gelen sollte im Sinne der vorliegenden Erfindung vorzugsweise im Bereich von 2 bis 30 Gew.-%, insbesondere bevorzugt im Bereich von 5 bis 15 Gew.-%, bezogen auf die Gesamtzusammensetzung liegen. Wird der Gehalt der Komponente (b) zu gering eingestellt, so ist ein stabiles Gel nicht herstellbar. Wird jedoch die Menge der Komponente (b) zu hoch gewählt, so ergibt sich eine nicht trockene Einstellung des Gels.

Im Sinne der vorliegenden Erfindung kann ein Teil des Polyoxyalkylen-Organopolysiloxans der allgemeinen Formel (I) durch die aus der EP 0 819 426 A2 bekannten $\alpha,\omega$-Polyetherpolysiloxane ersetzt werden. Insoweit wird voll inhaltlich auf die eingangs erwähnte Offenlegungsschrift Bezug genommen.

Besonders bevorzugt im Sinne der vorliegenden Erfindung ist es, 10 bis 50 Gew.-%, bezogen auf die Komponente (b) des Polyoxyalkylen-Organopolysiloxans der allgemeinen Formel (I) durch das $\alpha,\omega$-Polyethersiloxan der allgemeinen Formel (II) zu ersetzen.

Die erfindungsgemäßen Gele enthalten vorzugsweise 10 bis 80 Gew.-%, bezogen auf die Zusammensetzung an Wasser. Zur Einstellung des Brechungsindexes werden der wäßrigen Phase Alkohole und/oder Polyole zugesetzt. Besonders bevorzugt im Sinne der vorliegenden Erfindung ist die Anwesenheit von mindestens 5 Gew.-% und höchstens 78 Gew.-%, bezogen auf die Zusammensetzung oder mindestens 6 Gew.-% und bis zu 90 Gew.-% der Wasserphase, Alkohol und/oder Polyol, wobei diese Gruppen Ethanol, n-Propanol, i-Propanol, primäre, sekundäre und tertiäre aliphatische Alkohole mit mindestens einer und höchstens sechs Hydroxylgruppen und einer Kettenlange von 2 bis 6 Kohlenstoffatomen (Ethylenglycol, Glycerin, Propylenglycol bis Hexylenglycol, Polypropylenglycol, Sorbit und andere Zuckeralkohole), Polyglycerine mit einem Kondensationsgrad von 2 bis 10 (Diglycerin, Triglycerin) und Mischungen der Polyglycerine untereinander und mit Glycerinen umfassen.

Bei der Verwendung von UV-Absorbern in der Ölphase mit einer Gesamtmenge größer 1 %, bezogen auf die Zusammensetzung, werden Alkohole und/oder Polyole zum Einstellen des Brechungsindexes der hydrophilen (wäßrigen) Phase benötigt. Allein mit Wasser erreicht der Brechungsindex meist nicht den entsprechenden Wert der Ölphase. Es wurde überraschenderweise gefunden, daß diese aus Polyol, Wasser, Silikon und polaren UV-Absorbern zusammengesetzten transparenten Emulsionen temperatur- und langzeitstabil sind, da üblicherweise Emulsionen mit gemischtpolarer Ölphase und hohem Polyolgehalt schwierig zu stabilisieren sind.

Besonders bevorzugt im Sinne der vorliegenden Erfindung enthalten die transparenten Sonnenschutzgele UV-Absorber ausgewählt aus 4-Aminobenzoesäure, 3-(4'-Trimethylammonium)-benzyliden-bornan-2-on-methylsulfat, 3,3,5-Trimethylcyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo-[2,2,1]-heptan-1-methansulfonsäure) und ihre Salze, 1-(4-tert.-Butylphenyl)-3-(4-methoxyphenyl)propan-1,3-dion, 3-(4'-Sulfo)benzylidenbornan-2-on und Salze, 2-Cyan-3,3-diphenyl-acrylsäure(2-ethyl-hexylester) , 4-Bis (polyethoxy) aminobenzoesäure-polyethoxyethylester, 4-Dimethylaminobenzoesäure-2-ethyl-hexylester,Salicylsäure-2-ethylhexylester, 4-Methoxy-zimtsäureisoamylester, 4-Methoxy-zimtsäure-2-ethylhexylester, 2-Hydroxy-4-methoxy-benzophenon-5-sulfonsäure und das Natriumsalz, 3-(4'-Methylbenzyliden)-bornan-2-on, 3-Benzylidenbornan-2-on, 4-Isopropylbenzylsalicylat, 2,4,6-Trianilin-(p-carbo-2'-ethylhexyl-1'-oxy)-1,3,5-triazin, 3-imidazol-4-yl-acrylsäure und ihr Ethylester sowie N-([2 und 4]-[(2-Oxoborn-3-yliden)methyl]benzyl)acrylamid-Polymer oder deren Gemische.

Die Menge der wasserlöslichen UV-Absorber, die in den erfindungsgemäßen transparenten Sonnenschutzgelen enthalten sein können, sollte vorzugsweise 1 bis 10 Gew.-%, bezogen auf die Zusammensetzung, insbesondere

2 bis 8 Gew.-%, bezogen auf Zusammensetzung betragen.

Neben den oder anstelle der wasserlöslichen UV-Absorbern ist es selbstverständlich im Sinne der vorliegenden Erfindung auch möglich, öllösliche UV-Absorber einzusetzen.

Die Menge der öllöslichen Absorber richtet sich insbesondere auch nach dem Brechungsindex dieser Komponente, da zur Herstellung der erfindungsgemäßen transparenten Sonnenschutzgele die Wasserphase und die Silikonölphase den gleichen Brechungsindex aufweisen soll. Bevorzugterweise beträgt die Menge an löslichen UV-Absorbern 1 bis 10 Gew.-%, insbesondere 2 bis 8 Gew.-%, bezogen auf die Zusammensetzung.

Die Silikongelzusammensetzungen der vorliegenden Erfindung werden analog dem Stand der Technik, insbesondere der EP 0 568 102 A1 durch Zusammenmischung von Wasser, das den UV-Absorber in Lösung enthält, in die Komponenten (a) und (b) hergestellt. Der Gehalt an Wasser in der Silikongelzusammensetzung der vorliegenden Erfindung liegt vorzugsweise im Bereich von 0,2 bis 80 Gew.-% und insbesondere im Bereich von 0,3 bis 75 Gew.-%. Eine stabile Gelzusammensetzung kann nicht erhalten werden, wenn der Wassergehalt zu niedrig ist. Wird der Wassergehalt zu hoch gewählt, so trennt sich die Silikongelzusammensetzung und die Herstellung eines stabilen Silikongels wird außerordentlich schwierig.

Ausführungsbeispiele:

Die nachfolgend genannten Inhaltsstoffe von (A) und (B) wurden getrennt in der aufgeführten Reihenfolge unter Rühren zusammengefügt. Während gleichmäßigen Rührens bei Raumtemperatur wurde (B) langsam zu (A) gegeben. Anschließend wurde homogenisiert. Für klare Formulierungen müssen die Brechungsindices von (A) und (B) identisch sein. Dies konnte durch geringe Veränderung des Wasser/Glycerin-Verhältnisses erreicht werden. Die Mengenangaben in Gew.-% beziehen sich jeweils auf die Gesamtmenge.

Beispiel 1:

Die Formulierung wurde 3 Monate bei 45 °C, 2 1/2 Monate bei 60 °C und 5 mal bei -25 °C/Raumtemperatur getestet und zeigte in allen Fällen keine Separationserscheinungen.

| A: | |
|---|---|
| Cetyl Dimethicone-Copolyol (ABIL®EM 90) | ( 2 Gew.-%), |
| Cetyl Dimethicone (ABIL®Wax 9801) | ( 2 Gew.-%), |
| Cyclopentasiloxan und Cyclohexasiloxan (ABIL®B8839) | ( 6,5 Gew.-%), |
| Isopropylmyristat (TEGOSOFT®M) | ( 1 Gew.-%), |
| Octyldimethyl PABA (Eusolex®6007 Merck) | ( 0,5 Gew.-%), |
| Octyl-Methoxycinnamat (Parsol®MCX GIVAUDAN) | ( 4 Gew.-%), |
| B: | |
| Entionisiertes Wasser | (10 Gew.-%), |
| Natriumchlorid | ( 2 Gew.-%), |
| Glycerin | (72 Gew.-%). |

Es wurde ein stabiles Gel mit einem Lichtschutzfaktor LSF nach Colipa von 11 erhalten.

Beispiel 2:

Analog Beispiel 1 wurde unter Verwendung von

| A: | |
|---|---|
| Cetyl Dimethicone-Copolyol (ABIL®EM 90) | (2 Gew.-%), |
| Cetyl Dimethicone (ABIL®Wax 9801) | (2 Gew.-%), |
| Cyclopentasiloxan und Cyclohexasiloxan (ABIL®B8839) | (6,5 Gew.-%), |
| Isopropylmyristat (TEGOSOFT®M) | (1 Gew.-%), |
| Octyldimethyl PABA (Eusolex®6007 Merck) | (0,5 Gew.-%), |
| Octyl-Methoxycinnamat (Parsol®MCX GIVAUDAN) | (4 Gew.-%), |
| Butyl-Methoxydibenzoylmethan | (0,5 Gew.-%) |
| B: | |
| Entionisiertes Wasser | (20,95 Gew.-%) |
| Natriumchlorid | (2 Gew.-%) |
| Glycerin | (53 Gew.-%) |
| Phenylbenzimidazol-Sulfonsäure (Neo-Heliopan® Hydro (Haarmann & Reimer) | ( 7,8 Gew.-%) |
| Natriumhydroxid | ( 0,25 Gew.-%) |

wurde ein entsprechendes Gel hergestellt.

Der Sonnenschutzfaktor (Colipa) in-vivo des stabilen Gels wurde zu 16 bestimmt.

Beispiel 3:

Unter Verwendung eines ausschließlich wasserlöslichen Filters wurde ein Gel hergestellt:

| A: | |
|---|---|
| Cetyl Dimethicone-Copolyol (ABIL®EM 90) | ( 2 Gew.-%), |
| Cetyl Dimethicone (ABIL®Wax 9801) | ( 2 Gew.-%), |
| Cyclopentasiloxan und Cyclohexasiloxan (ABIL®B8839) | (11 Gew.-%), |
| Isopropylmyristat (TEGOSOFT®M) | ( 1 Gew.-%), |
| B: | |
| Entionisiertes Wasser | (27,75 Gew.-%), |
| Natriumchlorid | ( 2 Gew.-%), |
| Glycerin | (27 Gew.-%), |
| Phenylbenzimidazol-Sulfonsäure (30 Gew.-%) | (27 Gew.-%) |

und Rest NaOH.

Analog den vorgenannten Beispielen wurde auch hier ein stabiles Gel erhalten, der Sonnenschutztaktor (Colipa) in vivo wurde zu 18 bestimmt.

Die vorgenannten Produkte können als wasserresistent bezeichnet werden.

**Patentansprüche**

**1.** Transparente Sonnenschutzgele enthaltend

(a) Silikonöl

(b) Polyoxyalkylen-Organopolysiloxane der allgemeinen Formel (I)

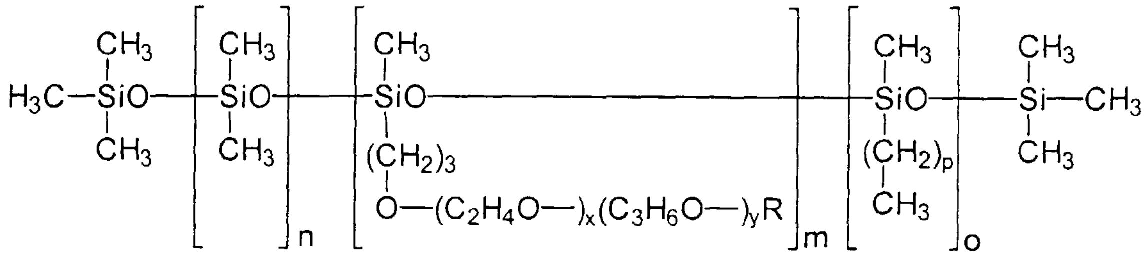

(I)

wobei

R ein Alkylrest mit 1 bis 4 Kohlenstoffatomen oder ein Wasserstoffrest ist,
n = 10 bis 200,
m = 1 bis 25 und
o = 1 bis 100 mit der Maßgabe ist, daß im durchschnittlichen Molekül o ≥ m und 3 o < n ist,
P = 7 bis 17 ist, und

die Molmasse des Restes $(C_2H_4O-)_x-(C_3H_6O-)_yR$ 250 bis 2000 beträgt, wobei x und y so gewählt sind, daß das Gewichtsverhältnis von Oxyethylen- zu Oxypropylengruppen 100 : 0 bis 20 : 80 beträgt,

(c) Wasser,

(d) eine Komponente ausgewählt aus Alkoholen und/oder Polyolen und

(e) wenigstens einen in der wäßrigen oder in der öligen Phase löslichen UV-Absorber.

**2.** Gele nach Anspruch 1, dadurch gekennzeichnet, daß das Silikonöl (a) ausgewählt ist aus Polysiloxan-Polyalkylen-Copolymeren, niedrig- bis hochviskosen Diorgano-Polysiloxanen wie Dimethyl-Polysiloxanen, Methylphenyl-Polysiloxanen und Dimethylsiloxan-Methylphenylsiloxancopolymeren, cyclischen Siloxanen wie Octamethyl-Cyclotetracyclosiloxan, Decamethyl-Cyclocentasiloxan und TetramethylTetraphenyl-Tetracyclosiloxan; Lösungen von cyclischen Siloxanen mit hoher Molmasse, von DimethylpolysiloxanGumen, Dimethylsiloxan-Methylphenylsiloxan-Copolymer-Gumen und Dimethylpolysiloxan-Gumen; Trimethylsiloxy-Kieselsäuren und cyclischen Siloxanlösungen von Trimethylsiloxy-Kieselsäuren, Diorgano-Polysiloxanen mit $C_6$ bis $C_{50}$ Alkylgruppen; und Aminogruppen enthaltenden Diorgano-Polysiloxanen.

**3.** Gele nach Anspruch 1 oder 2, dadurch gekennzeichnet, daß das Silikonöl (a) in einer Menge von 10 bis 80 Gew.-% enthalten ist.

**4.** Gele nach einem der Ansprüche 1 bis 3, dadurch gekennzeichnet, daß die Polyoxyalkylen-Organopolysiloxane der allgemeinen Formel (I) in einer Menge von 2 bis 30 Gew.-%, insbesondere 5 bis 15 Gew.-%, bezogen auf die Gesamtzusammensetzung enthalten sind.

**5.** Gele nach einem der Ansprüche 1 bis 4, dadurch gekennzeichnet, daß die Komponente (b) α,ω-Polyetherpolysiloxane der allgemeinen Formel (II)

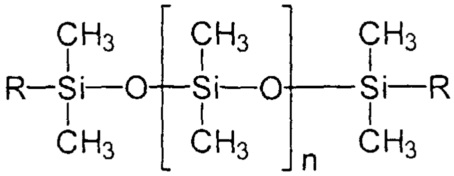

(II)

enthält, wobei

$R = -(CH_2-)_mO-(C_2H_4O-)_x(C_3H_6O-)_yR^1$
m = 2 bis 4,
x = 3 bis 100,
y = 0 bis 50
$R^1$ = H, $CH_3$,$CH_2CH_3$,
n = 50 bis 200,

und/oder Polyoxyalkylen-Organopolysiloxane der allgemeinen Formel (III):

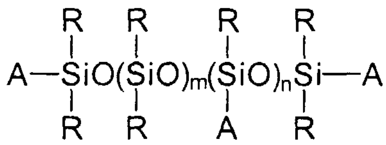

(III)

wobei

R für eine Methyl- oder Phenylgruppe steht,
A ausgewählt ist aus der Gruppe bestehend aus Methyl, Phenyl oder Poloxyalkylen-Gruppen der Formel

$$-C_3H_6O(C_2H_4O)_a(C_3H_6O)_bR',$$

wobei R' ausgewählt ist aus der Gruppe bestehend aus Wasserstoff, einer Acylgruppe oder einer Alkylgruppe mit 1 bis 4 Kohlenstoffatomen,
a eine ganze Zahl im Bereich von 5 bis 50
b eine ganze Zahl im Bereich von 5 bis 50,
m eine ganze Zahl im Bereich von 50 bis 1000 und
n eine ganze Zahl im Bereich von 1 bis 40 ist.

**6.** Gele nach Anspruch 5, dadurch gekennzeichnet, daß das $\alpha,\omega$-Polyethersiloxane der allgemeinen Formel (II) und/oder Polyoxyalkylen-Organopolysiloxan der allgemeinen Formel (III) in einer Menge von 10 bis 50 Gew.-%, bezogen auf die Komponente (b) enthalten ist.

**7.** Gele nach einem der Ansprüche 1 bis 6, dadurch gekennzeichnet, daß der Wassergehalt 10 bis 80 Gew.-%, bezogen auf die Gesamtzusammensetzung beträgt.

**8.** Gele nach einem der Ansprüche 1 bis 7, dadurch gekennzeichnet, daß der Gehalt an Alkoholen und/oder Polyolen der Komponente (d) 5 bis 78 Gew.-%, bezogen auf die Gesamtzusammensetzung oder 6 bis 90 Gew.-%, bezogen auf die Wasserphase beträgt.

**9.** Gele nach einem der Ansprüche 1 bis 8, dadurch gekennzeichnet, daß die Alkohole und Polyole ausgewählt sind aus Ethanol, n-Propanol, i-Propanol, primären, sekundären und tertiären aliphatischen Alkoholen mit mindestens einer und höchstens sechs Hydroxylgruppen und einer Kettenlänge von 2 bis 6 Kohlenstoffatomen, insbesondere Ethylenglycol, Glycerin, Propylenglycol bis Hexylenglycol, Polypropylenglycol, Sorbit, Polyglycerine mit einem Kondensationsgrad von 2 bis 10, insbesondere Diglycerin und Triglycerin sowie Mischungen der Polyglycerine untereinander und mit Glycerinen.

**10.** Gele nach einem der Ansprüche 1 bis 8, dadurch gekennzeichnet, daß die UV-Absorber ausgewählt sind aus 4-Aminobenzoesäure, 3-(4'-Trimethylammonium)-benzyliden-bornan-2-on-methylsulfat, 3,3,5-Trimethyl-cyclohexylsalicylac, 2-Hydroxy-4-methoxybenzophenon, 2-Phenylbenzimidazol-5-sulfonsäure und ihre Kalium-, Natrium- und Triethanolaminsalze,3,3'-(1,4-Phenylendimethin)-bis-(7,7-dimethyl-2-oxobicyclo-[2,2,1]-heptan-1-methansulfonsäure) und ihre Salze, 1-(4-terz.-Butylphenyl)-3-(4-methoxyphenyl)propan-1,3-dion, 3-(4'-Sulfo)benzylidenbornan-2-on und Salze, 2-Cyan-3,3-diphenyl-acrylsäure(2-ethyl-hexylester), 4-Bis(polyethoxy) aminobenzoesäure-polyethoxy-ethylester,4-Dimethylaminobenzoesäure-2-ethylhexylester, Salicylsäure-2-ethylhexylester,4-Methoxyzimtsäureisoamylester, 4-Methoxyzimtsäure-2-ethylhexylester, 2-Hydroxy-4-methoxy-benzophenon-5-sulfonsäure und

das Natriumsalz, 3-(4'-Methylbenzyliden)-bornan-2-on, 3-Benzylidenbornan-2-on,4-Isopropylbenzylsalicylat, 2,4,6-Trianilin-(p-carbo-2'-ethylhexyl-1'-oxy)-1,3,5-triazin,3-imidazol-4-yl-acrylsäure und ihr Ethylester sowie N-([2 und 4]-[(2-Oxoborn-3-yliden)methyl] benzyl)acrylamid-Polymer oder deren Gemische.

**11.** Gele nach einem der Ansprüche 1 bis 10, dadurch gekennzeichnet, daß die Menge der wasserlöslichen UV-Absorber 1 bis 10 Gew.-%, bezogen auf die Gesamtzusammensetzung beträgt.

**12.** Gele nach einem der Ansprüche 1 bis 11, dadurch gekennzeichnet, daß die Menge der öllöslichen UV-Absorber 1 bis 10 Gew.-%, bezogen auf die Gesamtzusammensetzung beträgt.